(19) Europäisches Patentamt
European Patent Office
Office européen des brevets

(11) **EP 3 221 444 B1**

(12) **EUROPEAN PATENT SPECIFICATION**

(45) Date of publication and mention
of the grant of the patent:
**02.03.2022 Bulletin 2022/09**

(21) Application number: **15861449.5**

(22) Date of filing: **17.11.2015**

(51) International Patent Classification (IPC):
**G01N 33/558** (2006.01)    **G01N 33/76** (2006.01)
**G01N 33/53** (2006.01)    **G16H 10/00** (2018.01)

(52) Cooperative Patent Classification (CPC):
**G01N 33/76; G01N 33/5306; G01N 33/558**

(86) International application number:
**PCT/US2015/061078**

(87) International publication number:
**WO 2016/081453 (26.05.2016 Gazette 2016/21)**

(54) **LATERAL FLOW ASSAY RATIO TEST**

LATERALFLUSSASSAYVERHÄLTNISTEST

TEST DE RAPPORT DE DOSAGE À ÉCOULEMENT LATÉRAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.11.2014 US 201462081138 P**

(43) Date of publication of application:
**27.09.2017 Bulletin 2017/39**

(73) Proprietor: **Siemens Healthcare Diagnostics Inc.
Tarrytown, NY 10591 (US)**

(72) Inventors:
• **UGRINOV, Krastyu
Granger, Indiana 46530 (US)**
• **SCHULMAN, Lloyd
Granger, Indiana 46530 (US)**
• **LEDDEN, David
Medway, Massachusetts 02053 (US)**
• **COX, Janine
Stoughton, MA 02072 (US)**
• **NOVAMO, Anne
Dedham, MA 02026 (US)**

(74) Representative: **Schweitzer, Klaus
Plate Schweitzer Zounek
Patentanwälte
Rheingaustrasse 196
65203 Wiesbaden (DE)**

(56) References cited:
WO-A1-00/70094    US-A- 5 807 755
US-A- 6 087 184    US-A1- 2007 172 963
US-A1- 2007 172 963    US-A1- 2012 015 429
US-A1- 2012 021 531

• LUND HANNE ET AL: "Epitope analysis and detection of human chorionic gonadotropin (hCG) variants by monoclonal antibodies and mass spectrometry.", TUMOUR BIOLOGY : THE JOURNAL OF THE INTERNATIONAL SOCIETY FOR ONCODEVELOPMENTAL BIOLOGY AND MEDICINE FEB 2014, vol. 35, no. 2, February 2014 (2014-02), pages 1013-1022, XP009500714, ISSN: 1423-0380
• CERVINSKI M A ET AL: "Qualitative point-of-care and over-the-counter urine hCG devices differentially detect the hCG variants of early pregnancy", CLINICA CHIMICA ACTA, ELSEVIER BV, AMSTERDAM, NL, vol. 406, no. 1-2, 11 August 2009 (2009-08-11), pages 81-85, XP026321577, ISSN: 0009-8981, DOI: 10.1016/J.CCA.2009.05.018 [retrieved on 2009-05-27]

(Cont. next page)

- **C. M. STURGEON ET AL: "Differences in Recognition of the 1st WHO International Reference Reagents for hCG-Related Isoforms by Diagnostic Immunoassays for Human Chorionic Gonadotropin", CLINICAL CHEMISTRY., vol. 55, no. 8, 18 June 2009 (2009-06-18), pages 1484-1491, XP055415255, WASHINGTON, DC. ISSN: 0009-9147, DOI: 10.1373/clinchem.2009.124578**
- **A. M. GRONOWSKI ET AL: "Characterization of the hCG Variants Recognized by Different hCG Immunoassays: An Important Step Toward Standardization of hCG Measurements", CLINICAL CHEMISTRY., vol. 55, no. 8, 1 August 2009 (2009-08-01), pages 1447-1449, XP055415073, WASHINGTON, DC. ISSN: 0009-9147, DOI: 10.1373/clinchem.2009.129205**

**Description**

**BACKGROUND**

The invention relates to determining the concentration of an analyte(s) in a liquid test sample by immunochromatography techniques-such as an immunochromatographic strip. Immunochromatographic strip formats are frequently used for qualitative, semi-quantitative and quantitative assays that use visual and reflectance based detection schemes. This type of immunoassay involves the application of a liquid test sample suspected of containing an analyte to be detected to an application site of an immunochromatographic test strip. The strip includes a matrix material through which the liquid test medium and analyte suspended or dissolved therein can flow by capillarity from the application site to one or more capture sites where a detectable signal, or the absence of such, reveals the presence of the analyte. Respective capture sites may refer to specific locations of the test strip impregnated with reagents. Color changes, readable by an instrument and/or the human eye, of the capture sites is an indication of the presence and concentration of analyte in the test fluid. In particular embodiments, the matrix material is fabricated from an absorbent material which allows the analyte and labeled antibodies specific thereto to flow through it along with the fluid test sample and to form analyte/labeled antibody conjugates which can be captured in specific capture zones thereof, to provide a detectable response. Examples of test fluid include, but are not limited to, urine, blood, plasma, and serum. US2007/172963 Ali discloses a lateral flow assay that encompasses a first region that contains a diffusibly bound labeled reagent complementary to a target analyte in the liquid sample, wherein the diffusibly bound labeled reagent and the target analyte form a diffusible first complex; a test region that contains a non-diffusibly bound capture reagent capable of complexing with the first complex; a control region that contains a non-diffusibly bound control reagent that is complementary to the diffusively bound labeled reagent; and a reference region that contains a non-diffusibly bound analyte capable of complexing with the diffusibly bound labeled reagent.

**SUMMARY OF THE INVENTIVE CONCEPT(S)**

In one aspect, the inventive concepts disclosed herein are directed to a lateral flow assay strip according to claim 1.
Additional aspects of the inventive concepts disclosed herein are directed to a method of determining a ratio of a second concentration to a first concentration according to claim 8.
Additional aspects of the inventive concepts disclosed herein are directed to a system according to claim 11.

**BRIEF DESCRIPTIONS OF THE DRAWINGS**

Figs. 1A and 1B depict an illustrative lateral flow assay strip.
Fig. 2 depicts another embodiment of a lateral flow assay strip.
Fig. 3 depicts yet another embodiment of a lateral flow assay strip.
Fig. 4 depicts another illustrative embodiment of a lateral flow assay strip.
Fig. 5 depicts an alternative embodiment of a lateral flow assay strip.
Fig. 6 depicts an illustrative embodiment of a reflectance-based optical detector instrument.

**DETAILED DESCRIPTION OF THE INVENTIVE CONCEPT(S)**

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. Also, it is to be understood that the phraseology and terminology employed herein is for the purpose of description and should not be regarded as limiting the inventive concepts disclosed and claimed herein in any way.
In the following detailed description of embodiments of the inventive concepts, numerous specific details are set forth in order to provide a more thorough understanding of the inventive concepts. However, it will be apparent to one of ordinary skill in the art that the inventive concepts within the instant disclosure may be practiced without these specific details. In other instances, well-known features have not been described in detail to avoid unnecessarily complicating the instant disclosure.
As used herein, the terms "comprises," "comprising," "includes," "including," "has," "having" or any other variation thereof, are intended to cover a non-exclusive inclusion. For example, a composition, a process, method, article, or apparatus that comprises a list of elements is not necessarily limited to only those elements but may include other elements not expressly listed or inherently present therein.

As used herein the terms "approximately," "about," "substantially" and variations thereof are intended to include not only the exact value qualified by the term, but to also include some slight deviations therefrom, such as deviations caused by measuring error, manufacturing tolerances, wear and tear on components or structures, settling or precipitation of cells or particles out of suspension or solution, chemical or biological degradation of solutions over time, stress exerted on structures, and combinations thereof, for example.

As used herein, the term "sample" and variations thereof is intended to include biological tissues, biological fluids, chemical fluids, chemical substances, suspensions, solutions, slurries, mixtures, agglomerations, tinctures, slides, powders, or other preparations of biological tissues or fluids, synthetic analogs to biological tissues or fluids, bacterial cells (prokaryotic or eukaryotic), viruses, single-celled organisms, lysed biological cells, fixed biological cells, fixed biological tissues, cell cultures, tissue cultures, genetically engineered cells and tissues, genetically engineered organisms, and combinations thereof, for example.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present). An inclusive or may be understood as being the equivalent to: at least one of condition A or B.

In addition, use of the "a" or "an" are employed to describe elements and components of the embodiments herein. This is done merely for convenience and to give a general sense of the inventive concepts. This description should be read to include one or at least one and the singular also includes the plural unless it is obvious that it is meant otherwise.

Finally, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

The invention relates to determining the concentration of an analyte(s) in a liquid test sample by immunochromatography techniques-such as an immunochromatographic strip. Immunochromatographic strip formats are frequently used for qualitative, semi-quantitative and quantitative assays that use visual and reflectance based detection schemes. This type of immunoassay involves the application of a liquid test sample suspected of containing an analyte to be detected to an application site of an immunochromatographic test strip. The strip includes a matrix material through which the liquid test medium and analyte suspended or dissolved therein can flow by capillarity from the application site to one or more capture sites where a detectable signal, or the absence of such, reveals the presence of the analyte. Respective capture sites may refer to specific locations of the test strip impregnated with reagents. Color changes, readable by an instrument and/or the human eye, of the capture sites is an indication of the presence and concentration of analyte in the test fluid. In particular embodiments, the matrix material is fabricated from an absorbent material which allows the analyte and labeled antibodies specific thereto to flow through it along with the fluid test sample and to form analyte/labeled antibody conjugates which can be captured in specific capture zones thereof, to provide a detectable response. Examples of test fluid include, but are not limited to, urine, blood, plasma, and serum.

An illustrative analyte of interest is human chorionic gonadotropin (hCG) and the variants thereof. Detection of hCG hormone in liquid test samples is a leading diagnostic test for pregnancy. Physiologically, hCG molecules exist within the human body in different metabolic states referred to as 'hCG variants.' Illustrative diagnostic tests may aim to detect a group of multiple hCG variants in a liquid sample in order to facilitate the determination of the total concentration of the hCG variants-this concentration may be referred to as 'total hCG' (hCGt). While certain illustrative diagnostic tests may, in fact, test for all hCG variants it should be understood that other illustrative diagnostic tests may only test for a subset of hCG variants. This subset of hCG variants can be selected in order to approximate the actual hCGt concentration in the liquid sample without actually testing for all hCG variants.

Each variant of hCG has a specific physiological function(s) whose expression varies with, for example, the stage of a pregnancy or the developing of a malignant formation. For example, illustrative variant hyperglycosylated hCG (hCGh) can be an important indicator in evaluating early pregnancy development (i.e., implantation), early pregnancy loss (EPL), and malignancies of the reproductive system. The uses of a hCGh specific antibody (such as monoclonal antibody B-152) allows for the discrete evaluation of the hCGh expression patterns from within the larger pool of total hCG-thus enabling the concentration of hCGh within a liquid sample to be determined.

Once the respective concentrations of hCGt and hCGh are determined, a ratio of hCGh to hCGt can be computed. This hCGh/hCGt ratio can be used, for example, to predict the outcome of pregnancies as well as the success of in vitro fertilization procedures.

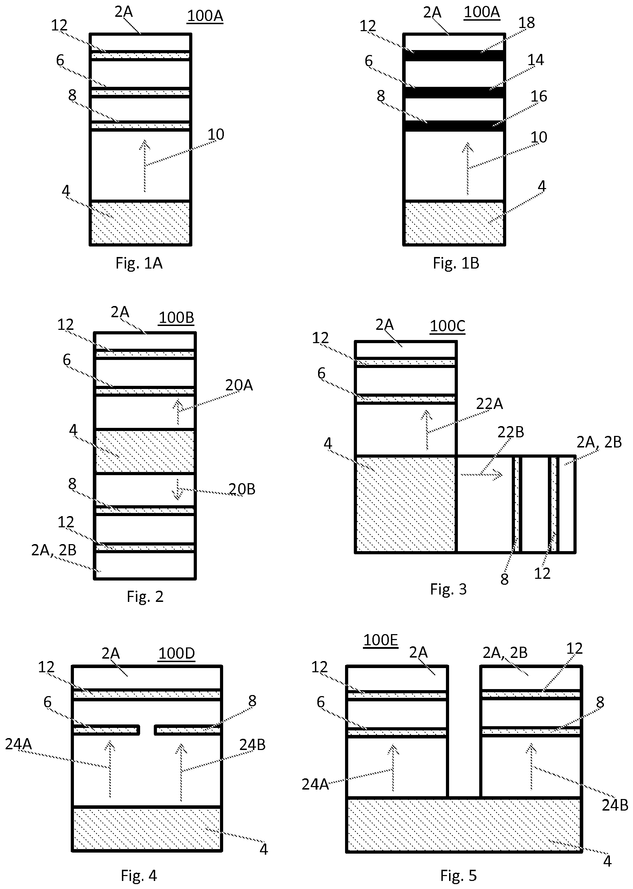

Turning now to Figs. 1A-5, Figs. 1A and 1B depict a lateral flow assay strip 100A configured to detect two analyte concentrations: the concentration of hCGh and the concentration of hCGt. Fig. 1A represents lateral flow assay strip 100A before the sample liquid has been applied. Fig. 1B provides an illustrative example of the lateral flow assay strip of Fig. 1A after a sample liquid has been applied and allowed to flow through the substrate 2A along flow path 10.

Assay strip 100A is comprised of one or more chromatographic substrates 2A. Chromatographic substrates 2A, 2B may include any matrix material through which the liquid test medium and analyte suspended or dissolved therein can flow by capillarity. Chromatographic substrates 2A, 2B comprise a sample application site 4 for application of a portion of the liquid sample. Sample application site can be comprised of, for example, one or more of: a region on a respective chromatographic substrate 2A, 2B, a sample pad, or conjugate pad. Illustrative sample pads may be for receiving a sample application, sample treatment, and sample filtration. Illustrative conjugate pads may contain colloidal gold particles or colored latex particles (e.g., a label) coated with anti-beta hCG monoclonal antibody which mix with the sample liquid upon application of the sample liquid to the sample application site 4. The sample application site 4 should be located a distance from each of the capture sites such that the sample liquid flows from the sample application site 4 towards each capture site(s). For example, the sample application site 4 of the assay strip 100A in Fig. 1 is located on a proximal end of a first surface of the assay strip 100A.

Lateral flow assay strip 100A further contains a first capture site 6 disposed on the first surface of a first chromatographic substrate 2A. The first capture site 6 is spaced apart from the application site 4 such that when the liquid sample is applied to the sample application site 4, the liquid sample moves through the first chromatographic substrate 2A from the sample application site 4 to the first capture site 6. The first capture site 6 comprises an anti-hCG capture antibody with broad affinity for the group of multiple hCG variants (as described above) which are collectively referred to as hCGt. For example, first capture site 6 may be a region of anti-hCG antibody, such as a goat polyclonal anti-hCG antibody. Once sample liquid, moving through the substrate 2A, reaches the first capture site 6, the presence of the hCG variants in the liquid sample is indicated by the presence of a first detectable signal 14 caused by the formation of an antibody-hCG-antibody-colloidal gold particle (e.g., a 'label') complex at the first capture site 6. The first detectable signal 14 is a color change of the first capture site 6. The first detectable signal 14 can be visually measured by an individual or determined using an instrument which measures the amount of light reflected by the complex at the first capture site 6 (e.g., measures the detectable signal 14). The more intense the color of the first detectable signal 14, the higher the concentration of total hCG in the sample. It should be appreciated that while the first capture site 6 and the first detectable signal 14 are illustrated in the Figures as straight lines, they may take on a variety of other shapes and configurations.

Lateral flow assay strip 100A further contains a second capture site 8 that can be located on the first surface of the first chromatographic substrate 2A-along with the first capture site 6-or a second, separate chromatographic substrate 2B-as will be described below in connection with Figs. 2-5. The second capture site 8 is spaced apart from the sample application site 4 such that when the liquid sample is applied to the sample application site 4, the liquid sample moves from the sample application site 4 to the second capture site 8 through the first chromatographic substrate 2A or the second chromatographic substrate 2B. The second capture site 8 comprising an anti-hCG capture antibody-known to those skilled in the art-that is specific to a variant of hCG such as the hCGh variant of hCG. Once sample liquid, moving through the substrate 2A, 2B, reaches the second capture site 8, the presence of the hCGh variant in the liquid sample is indicated by the presence of a second detectable signal 16 caused by the formation of an antibody-hCGh-antibody-colloidal gold particle (e.g., a 'label') complex at the second capture site 8. The second detectable signal 16 is a color change of the second capture site 8. The second detectable signal 16 can be visually measured by an individual or determined using an instrument which measures the amount of light reflected by the complex at the second capture site 8 (e.g., measures the second detectable signal 16). The more intense the color of the second capture site 8 the higher the concentration of the hCGh variant.

The lateral flow assay strip 100A may also be provided with a control site 12 located on the first surface. Control site 12 may comprise of one or more anti-species antibodies-such as goat anti-mouse IgG, goat anti-donkey IgG, or donkey IgG label-impregnated into the substrate 2. The control site 12 may be located past the capture sites 6, 8 in the direction of sample liquid flow. In other words, one or both of capture sites 6, 8 are located in between the control site 12 and the sample application site 4. Appearance of control detectable signal 18 confirms that the correct procedures were used. Thus, it confirms that sample was added, it mixed with and solubilized the colored particle dried to the pad and the complex flowed through the membrane resulting in binding of the colored particle to at the control site 12. It should be understood that lateral flow assay strip 100A may further contain additional sites as well-such as a reference site (the absence of a signal at which indicates the test results are invalid).

Figs. 1A and 1B depict a lateral flow assay strip 100A in which the first capture site 6 and the second capture site 8 are disposed on the first chromatographic substrate 2A. In this configuration, liquid sample flowing along flow path 10, from the sample application site 4 towards the first and second capture sites 6, 8, and passes through the second capture site 8 before passing through the first capture site 6.

In the example of Fig. 1B, the first detectable signal 14, the second detectable signal 16, and the control detectable signal 18 have appeared. The intensity of the first detectable signal 14 represents the concentration of hCGt (excluding hCHh) in the sample liquid, The intensity of second detectable signal 16 represents the concentration of hCGh in the sample liquid. The presence of the control line detectable signal 18 indicates that the sample was added, it mixed with and solubilized the colored particle dried to the pad and the complex flowed through the substrate 2A resulting in binding of the colored particle at the control site 12.

Figs. 2-5 all represent illustrative embodiments in which liquid sample flowing from the sample application site 4 along a flow path passes through either of the first capture site 6 or the second capture site 8-but not both. Fig. 2 depicts an alternative configuration of a lateral flow assay strip 100B in which the sample application site 4 is located between the first capture site 6 and the second capture site 8 which are located at the distal end and the proximal end of the first surface of the substrate 2A, 2B, respectively. In the configuration of Fig. 2, liquid sample applied to the sample application site flows outwardly from the sample application site 4 along both of two flow paths 20A, 20B towards the first capture site 6 and the second capture site 8, respectfully. Flow paths 20A, 20B are parallel to one another but are oriented 180 degrees to one another. Liquid sample moving along respective flow paths 20A, 20B pass through one, but not both, of the first capture site 6 or the second capture site 8.

Fig. 3 depicts yet another embodiment of a lateral flow assay strip 100C. In Fig. 3 liquid sample applied to the sample application site flows outwardly along two distinct flow paths 22A, 22B. The two flow paths 22A, 22B are oriented at an angle to one another and directed towards one of the first capture site 6 and the second capture site 8, respectfully. While in Fig. 3 the two paths 22A, 22B are depicted as forming a 90 degree angle to one another it should be understood that this angle can be set to a value of anywhere from 1 to 180 degrees (such as, for example, approximately 15, 25, 45, 75, 90, 115, or 145 degrees) so long as flow paths 22A, 22B remain distinct. Flow paths 22A, 22B can be considered distinct as long as liquid sample flowing from the sample application site 4 towards capture sites 6, 8 passes through one, but not both, of the first capture site 6 or the second capture site 8. The first capture site 6 and the second capture site 8 may be located on the first surface of a common substrate 2A, 2B or on the first surfaces of separate substrates 2A, 2B. For example, the substrate 2A in Fig. 3 may be cut into an 'L' shape such that first capture site 6 and the second capture site 8 are on the same substrate 2A. Alternatively, first capture site 6 and the second capture site 8 may be on separate substrates 2A, 2B that have adjoining sample application pads or a common sample application pad. This allows liquid sample applied to the sample application site 4 to flow toward both the first capture site 6 and the second capture site 8.

Fig. 4 illustrates another alternative configuration for a lateral flow assay strip 100D in which the first capture site 6 and the second capture site 8 are disposed on opposite sides of the first surface of a single chromatographic substrate 2A and in between the same sample application site 4 and the same control site 12. In this configuration, liquid sample will flow from the proximal to the distal end of the substrate 2A along (1) flow path 24A toward the first capture site 6 and (2) flow path 24B toward the second capture site 8. However, liquid flowing along parallel flow paths 24A and 24B, respectively, will pass through one, but not both, of first capture site 6 and the second capture site 8. As shown in Fig. 4, an embodiment of the assay strip 100D has the first capture site 6 and the second capture site 8 located a uniform distance from the sample application site 4. In this configuration, the liquid sample, flowing along flow path 24 from the sample application site toward the first and the second capture sites 6, 8, reaches both the first capture site 6 and the second capture site 8 at approximately the same time. In other variations of the embodiment of Fig. 4, the spacing between (1) the first capture site 6 and the sample application site 4 and (2) the second capture site 8 need not be equal (e.g., the first capture site 6 may be closer or further from the sample application site 4 than the second capture site 8).

Figure 5 depicts a 'U' shaped lateral flow assay 100E that functions in a manner similar to that of the embodiments described with respect to Fig. 4. The most notable difference being that the substrate(s) 2A, 2B of lateral flow assay 100E has a 'U' shaped cut-out between the first and second capture sites 6, 8. The 'U' shaped cut-out has a closed end located near the proximal end of the substrate(s) 2A, 2B and an open end located at the distal end of the substrate(s) 2A, 2B. The first capture site 6 and the second capture site 8 may be located on a common substrate 2A or on separate substrates 2A, 2B. For example, the substrate 2A in Fig. 5 may be cut into a 'U' shape such that first capture site 6 and the second capture site 8 are on the first surface of the same substrate 2A. Alternatively, first capture site 6 and the second capture site 8 may be on the first surfaces of separate substrates 2A, 2B that have adjoining sample application pads or a common sample application pad between them. This allows liquid sample applied to the sample application site 4 to flow toward both the first capture site 6 and the second capture site 8.

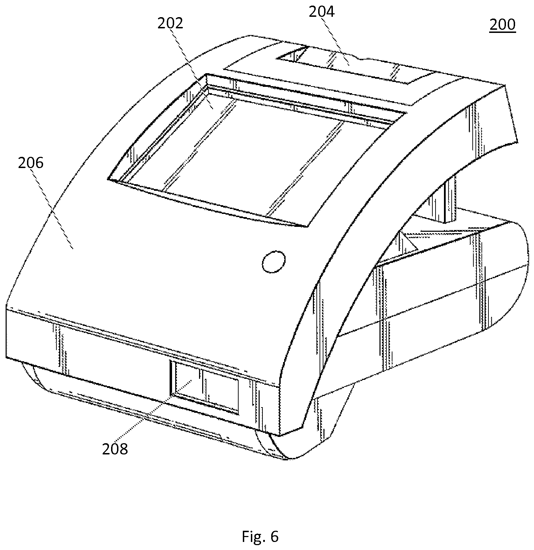

The illustrative embodiments of the lateral flow assays 100A through 100E depicted in Figs. 1A through 5-as well as those obvious variations of which-(collectively referred to as 'lateral flow assays 100') can be interpreted to determine the ratio of hCGt to hCGh in the liquid sample using an optical detector instrument 200 (an example of which is shown and described in connection with Fig. 6) or visually by an individual without the aid of the instrument 200. Illustrative methods of evaluating the above described lateral flow assays 100A-E without the use of optical detector instrument 200 include viewing any one of the above lateral flow assays 100A-E after the liquid sample has been applied to the at least one sample application site and allowed to move through the chromatographic substrate from the at least one sample application site towards the first capture site and the second capture site. The concentration of hCGt variants and hCGh variant may then be obtained at the first capture site and the second capture site by visually evaluating the intensity of the first detectable signal and the second detectable signal, respectively. The visual evaluation of the first detectable signal and the second detectable signal can be done, for example, by visually comparing (1) the relative intensity of the first detectable signal 14 against the second detectable signal 16 (e.g., determining whether the detectable signals have the same or different intensity) and/or (2) the detectable signals 14, 16 against one or more reference sources. For example, a reference source may contain reference values which each visually approximate known concentration of hCGt and hCGh at the first capture site 6 and the second capture site 8 on the lateral flow assay 100 being used. For example, reference values in the reference source may be in the form of color samples which replicate the color and intensity of the first detectable signal 14 and the second detectable signal 16 will exhibit should the liquid sample contain corresponding concentrations of hCGt and hCGh, respectively.

The ratio of the concentration of the hCGh variant in the liquid sample to the total concentration of all hCG variants (e.g., hCGt) in the liquid sample may then be determined based on the obtained concentration of hCGt variants at the first capture site and the obtained concentration of the hCGh variant at the second capture site. The manner in which the ratio of hCGh to hCGt can be computed varies on the configuration of the lateral flow assay 100. For example, where the lateral flow assay is configured so that liquid sample flowing from the sample application site 4 passes through either the first capture site 6 or the second capture site 8-but not both (as in lateral flow assays 100B through 100E)-the ratio can be computed by simply dividing the obtained concentration of hCGh by the obtained concentration of hCGt. The ratio can also be visually computed by visually comparing the intensity of the first detectable signal 14 against the intensity of the second detectable signal 16 with or without the aid of a reference source.

Alternatively, when the liquid sample, moving through the first chromatographic substrate, moves through the second capture site 8 and then first capture site 6-as is the case with the embodiment described in connection with Fig. 1-the ratio may be computed differently. Because the second capture site-which is hCGh specific-captures hCGh, the liquid sample passing through the first capture site will have been partially-or completely-depleted of hCGh. The actual concentration of hCGt in the liquid sample may then be computed by calculating the product of the obtained concentration of hCGh (R1) at the second capture site 8 and the obtained concentration of hCGt (R2) at the first capture site 6. The ratio can then be computed by dividing the obtained concentration of hCGh by the actual concentration of hCGt. This equation is shown below.

$$\text{hCGh/hCGt ratio} = (R1)/(R1+R2) \quad \text{(Equation 1)}.$$

In yet another illustrative example, the ratio can be determined by comparing the intensities of the first detectable signal 14 and the second detectable signal 16 of any one of lateral flow assays 100A-100E against a reference table. The reference table may index one or more hCGh/hCGt ratios-listed in individual cells- as a function of corresponding hCGh concentrations and hCGt concentrations-respectively listed in a first column and first row of the reference table. Using the reference chart, an individual can use hCGh concentrations and hCGt concentrations-determined using a reference source-to find the corresponding hCGh/hCGt ratio. Alternatively, an illustrative reference chart may index one or more hCGh/hCGt ratios-listed in individual cells of the reference chart- as a function of reference values for hCGh concentrations and hCGt concentrations (as described above) respectively depicted in a first column and first row of the reference table. This configuration may allow a user to determine an hCGh/hCGt ratio by matching the first detectable signal 14 and the second detectable signal 16 with reference color/intensity values in the first row/column of reference table.

While the illustrative embodiments of lateral assay strips 100 described above relate to hCGh and hCGt, the above described lateral flow assays can also be used to determine the ratio between hCGt and other variants. For example, lateral assay strips 100 can be used to compute the ratio of hCGt to hCG beta core fragment ('hCGbcf). Formed when urine is processed by the kidneys, too high of a concentration of hCGbcf in urine may invalidate a test result. This "high dose hook effect" often results in false negatives-putting patients at high risk of improper treatment. The embodiments of the lateral flow assays 100 above can be adapted to test for the hCGt to hCGbcf ratio by replacing the anti-hCG capture antibody of the above described second capture site 8 with an anti-hCG capture antibody-known to those skilled in the art-that is specific to the hCGbcf variant of hCG. Depending on the configuration of the lateral flow assay 100, the hCGt to hCGbcf ratio can be computed in the manner described above and below.

It should be further understood that, while the illustrative embodiments of lateral assay strips 100 described above relate to variants of hCG, the above described lateral flow assays can also be used to determine the ratio between any two analytes-or variants thereof-present in the liquid sample and is not limited to hCG and the variants thereof.

While the lateral flow assays 100A-100E shown and described above are strips, it should be appreciated by one of skill in the art that a lateral flow assay may also be incorporated into a cassette structure. An illustrative cassette may be a disposable, single-use cassette for doing a lateral flow immunoassay tests, for example, in the conventional manner. For example, the cassette may integrate the lateral flow assay into a housing which is comprised of a top piece, which defines a window, and a bottom piece. The cassette may have an opening or well in the top piece into which a fluid sample, such as urine, is placed so as to come into contact with the lateral flow assay. The housing may contain another window through which the first and second detectable signals 14, 16 can be viewed.

Embodiments of the lateral flow assay, including those lateral flow assays 100A-E as shown and described above, may be interpreted by a reflectance-based optical detector instrument 200. An illustrative example of such an instrument 200 is a reflectance spectroscope, or "reflectometer," which is shown in Fig. 6. Instrument 200 is for optically inspecting lateral flow assays after liquid samples have been placed thereon. The particular optical inspection instrument 200 shown in Fig. 6 is representative of a CLINITEK STATUS® Urine Chemistry Analyzer available from Siemens Healthcare Diagnostics, of Tarrytown, N.Y.. As will be appreciated by those skilled in the art, the present invention could be implemented in other reflectance-based instruments and is not restricted to embodiments provided herein

Instrument 200 may include an input and output device in the form of a touch screen 202. An output port 204 may be provided as a means for printing a report (e.g., test or diagnostic report) to an operator or user of machine 200. As will also be appreciated by those skilled in the art, other forms of input and output mechanisms may be used. For example, Instrument 200 may be configured to couple, by wired or wireless means, to an external computer, handheld computer, network, monitor, printer, audio/visual system or the like. A housing 206 houses the touch screen 202, as well as a variety of internal functional elements. An input port 208 is provided to facilitate insertion of one or more lateral flow assays 100 via a carriage (not shown).

Instrument 200 further comprises an optical sensor for the optical inspection of lateral flow assays 100, a non-transitory computer-readable storage medium on which computer-executable instructions are stored, and a processor. While not shown in Fig. 6, it should be understood that the optical sensor, computer-readable storage medium, and the processor are disposed inside housing 206. Processor may be operatively coupled to the optical sensor and the computer-readable storage medium. Processor may also be coupled with external computing platforms or data storage devices in order to send/receive information.

Once a portion of the liquid sample has been allowed to flow from the sample application site 4 to the capture sites 6, 8 of a lateral flow assay 100, the Instrument 200 can be used to analyze the first detectable signal 14 and the second detectable signal 16. For example, the optical sensor-in cooperation with the Processor and/or the computer-executable instructions-may measure the first and second detectable signals 14, 16 by measuring the characteristics of the light which is reflected (i.e., the reflectance) by the complexes at the respective first capture site 6 and the second capture site 8. The results of this measurement are then used by the processor to determine the corresponding hCGt and hCGh concentrations, respectively. The processor can identify the corresponding hCGt and hCGh concentrations by comparing the measured characteristics of the first and second detectable signals 14, 16 to reference data stored in the computer-readable storage medium. As one of skill in the art will appreciate, the reference data may comprise reference measurements that are correlated to known concentrations of hCGt and hCGh. By finding a reference measurement that corresponds to a measured characteristic, the processor can determine the concentrations of hCGt and hCGh in the liquid samples. The processor may then output the determined detectable signals 14, 16 to an output device (such as screen 202 or printer 204), an external computing platform, or a data storage device. The processor may also compute the hCGh/hCGt ratio, as described above, using the determined hCGt and hCGh concentrations and output the same. Alternatively, when the processor has outputted the determined detectable signals 14, 16 to an external computing device, that external device may use the received detectable signals 14, 16 to compute the hCGh/hCGt ratio, as described above.

Processor may have any suitable architecture, such as a general processor, central processing unit, digital signal processor, application specific integrated circuit, field programmable gate array, digital circuit, analog circuit, combinations thereof, or any other now known or later developed device for processing data. Likewise, processing strategies may include multiprocessing, multitasking, parallel processing, and the like. A program may be uploaded to, and executed by, the processor. The processor implements the program alone or includes multiple processors in a network or system for parallel or sequential processing.

The processor may perform the workflows, machine learning, model training, model application, and/or other processes described herein. For example, the processor or a different processor is operable to extract terms for use in modeling, learn a probabilistic model, and apply the trained probabilistic model. For applying the model, the model may have been trained by a different processor or the same processor. The processor outputs the state and/or associated information on the display, into a memory, over a network, to a printer, or in another media. The display is text, graphical, or other display.

The processor operates pursuant to instructions. The instructions and/or patient records for training a probabilistic model or for inferring a medical concept from a medical transcript are stored in a computer readable memory such as an external storage, ROM, and/or RAM. The instructions for implementing the processes, methods and/or techniques discussed herein are provided on computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system. Because some of the constituent system components and method acts depicted in the accompanying figures may be implemented in software, the actual connections between the system components (or the process steps) may differ depending upon the manner of programming.

Additionally, methods of using the lateral flow assays 100, as described above, may include the steps of providing or teaching an individual with some or all of the necessary information and/or materials described above. An illustrative method for determining a ratio of hCGh to hCGt in a liquid sample may comprise the following steps; teaching the step of viewing any of the lateral flow assays 100 the liquid sample has been applied to the at least one sample application site such that the liquid sample has moved through the chromatographic substrate 2A, 2B from the at least one sample application site 4 towards the first capture site 6 and the second capture site 8; teaching the step of obtaining the concentration of total hCG variants at the first capture site 6 by evaluating the first detectable signal 14; teaching the step of obtaining the concentration of the hCGh variant at the second capture site 8 by evaluating the second detectable signal 16; and teaching the step of determining the ratio of the concentration of the hCGh variant in the liquid sample to the total concentration of all hCG variants in the liquid sample based on the obtained concentration of all hCG variants at the first capture site 6 and the obtained concentration of the hCGh variant at the second capture site 8. Illustrative methods may further include: providing or teaching the use of a first reference value, the first reference value corresponding to a known concentration of all hCG variants in a first reference liquid sample; and providing or teaching the use of a second reference value, the second reference value corresponding to a known concentration of the hCGh variant in a second reference liquid sample. The step of obtaining the concentration of all hCG variants at the first capture site may also comprise teaching the step of comparing the first detectable signal against the first reference value. The step of obtaining the concentration of the hCGh variant at the second capture site may also comprise teaching the step of comparing the second detectable signal against the second reference value.

## Claims

1. A lateral flow assay strip comprising:

   one or more chromatographic substrates;
   a sample application site on each chromatographic substrate for application of a portion of a liquid sample;
   a first capture site disposed on a first chromatographic substrate, the first capture site being spaced apart from the application site such that when the liquid sample is applied to the sample application site the liquid sample moves through the first chromatographic substrate from the sample application site to the first capture site, the first capture site comprising an anti- human chorionic gonadotropin ('hCG') capture antibody with broad affinity for a group of multiple hCG variants, the presence of the group of hCG variants in the liquid sample being indicated at the first capture site by the presence of a first detectable signal caused by the presence of sandwich complexes at the first capture site; and
   a second capture site disposed on the first chromatographic substrate or a second chromatographic substrate, the second capture site being spaced apart from the sample application site such that when the liquid sample is applied to the sample application site, the liquid sample moves through the first chromatographic substrate or a second chromatographic substrate from the sample application site to the second capture site, the second capture site comprising an anti-hCG capture antibody that is specific to a first variant of hCG, the presence of the first variant of hCG in the liquid sample being indicated at the second capture site by the presence of a second detectable signal caused by the presence of the sandwich complexes at the second capture site.

2. The lateral flow assay strip of claim 1, wherein the first capture site and the second capture site are disposed on the first chromatographic substrate.

3. The lateral flow assay strip of claim 1, wherein the lateral flow assay contains a first substrate and a second substrate, the first substrate comprising the first capture site and the second substrate comprising the second capture site.

4. The lateral flow assay strip of any of claims 1 and 2, wherein first capture site and the second capture site are disposed on a first chromatographic substrate along a first liquid sample flow path, the first liquid flow path extending from a first sample application site toward both of the first and the second capture sites.

5. The lateral flow assay strip of any of claims 1 to 3, wherein liquid sample moving through the first chromatographic substrate reaches the second capture site before reaching the first capture site.

6. The lateral flow assay strip of any of claims 1 to 3, wherein liquid sample moving through the first chromatographic substrate reaches the first capture site and the second capture site at approximately the same time.

7. The lateral flow assay strip of any of claims 1 to 2, wherein the first capture site is disposed on a first surface of the first chromatographic substrate along a second liquid sample flow path extending from the sample application site to the first capture site , wherein the second capture site is disposed on the first surface of the first chromatographic substrate along a third liquid sample flow path, wherein the second liquid sample flow path does not extend through the second capture site, wherein the third liquid sample flow path does not extend through the first capture site.

8. A method of determining a ratio of a second concentration to a first concentration comprising:

viewing the lateral flow assay strip of any of claims 1 to 3 after a liquid sample has been applied to the at least one sample application site such that the liquid sample has moved through the chromatographic substrate from the at least one sample application site towards the first capture site and the second capture site;
obtaining a first concentration of a group of multiple hCG variants at the first capture site by evaluating the first detectable signal;
obtaining a second concentration of a first hCG variant at the second capture site by evaluating the second detectable signal; and
determining the ratio of the second concentration to the first concentration.

9. The method of claim 8, wherein the step of obtaining the first concentration comprises comparing the first detectable signal against a first reference value, the first reference value corresponding to a known concentration of the group of multiple hCG variants in a first reference liquid sample in a corresponding lateral flow assay; and
wherein the step of obtaining the second concentration comprises comparing the second detectable signal against a second reference value, the second reference value corresponding to a known concentration of the first hCG variant in a second reference liquid sample in the corresponding lateral flow assay.

10. The method of claim 9, wherein liquid sample moving through the first chromatographic substrate reaches the second capture site before reaching the first capture, and wherein determining the ratio of the second concentration of the first variant ($R_1$) in the liquid sample to the first concentration of the group of hCG variants ($R_2$) in the liquid sample comprises using the equation:

$$(R1)/(R1+R2).$$

11. A system comprising an optical detector and a non-transitory computer-readable medium that stores instructions which, when executed by the system, causes the system having at least one processor to interpret the results of the lateral flow assay strip of any of claims 1 to 3, after the liquid sample has been applied to the at least one sample application site such that the liquid sample has moved through the chromatographic substrate from the at least one sample application site towards the first capture site and the second capture site, by:

acquiring the concentration of a group of multiple hCG variants at the first capture site and the concentration of a first variant at the second capture site by obtaining the respective first detectable signal and the second detectable signal, wherein the first detectable signal and the second detectable signal are obtained by measuring the reflectance of the first capture site and the second capture site, respectively, with the optical detector; and
determining the ratio of the concentration of the first hCG variant in the liquid sample to the concentration of the group of multiple hCG variants in the liquid sample based on the obtained concentration of the group of multiple hCG variants at the first capture site and the concentration of the first hCGh variant at the second capture site.

12. The system of claim 11, wherein the step of obtaining the concentration of the group of hCG variants at the first capture site comprises comparing the first detectable signal against a first reference value, the first reference value corresponding to a known concentration of the group of hCG variants in a first reference liquid sample; and
wherein the step of obtaining the concentration of the first hCG variant at the second capture site comprises comparing the second detectable signal against a second reference value, the second reference value corresponding to a known concentration of the first hCG variant in a second reference liquid sample.

13. The system of claim 12, wherein liquid sample moving through the first chromatographic substrate reaches the second capture site before reaching the first capture site (e.g., the device of claim 4), and wherein determining the ratio of the concentration of the first variant ($R_1$) in the liquid sample to the total concentration of the group of hCG variants ($R_2$) in the liquid sample comprises using the equation:

$$(R1)/(R1+R2).$$

**Patentansprüche**

1. Lateral-Flow-Teststreifen, umfassend:

   ein oder mehrere chromatographische Substrate;
   eine Probenauftragstelle auf jedem chromatographischen Substrat zum Auftragen eines Teils einer Flüssigprobe;
   eine erste Einfangstelle, die auf einem ersten chromato-graphischen Substrat angeordnet ist, wobei die erste Einfangstelle von der Auftragstelle so beabstandet ist,
   dass die Flüssigprobe, wenn sie auf die Probenauftragstelle aufgetragen wird, von der Probenauftragstelle durch das erste chromatographische Substrat zur ersten Einfangstelle zieht, wobei die erste Einfangstelle einen Einfang-antikörper gegen menschliches Choriongonadotropin ('hCG') mit breiter Affinität für eine Gruppe von mehreren hCG-Varianten umfasst, wobei das Vorliegen der Gruppe von hCG-Varianten in der Flüssigprobe an der ersten Einfangstelle durch das Vorliegen eines ersten nachweisbaren Signals angezeigt wird, das durch das Vorliegen von Sandwich-Komplexen an der ersten Einfangstelle verursacht wird; und
   eine zweite Einfangstelle, die auf dem ersten chromatographischen Substrat oder einem zweiten chromato-graphischen Substrat angeordnet ist, wobei die zweite Einfangstelle von der Probenauftragstelle so beabstandet ist, dass die Flüssigprobe, wenn sie auf die Probenauftragstelle aufgetragen wird, von der Probenauftragstelle durch das erste chromatographische Substrat oder ein zweites chromatographisches Substrat zur zweiten Einfangstelle zieht, wobei die zweite Einfangstelle einen Anti-hCG-Einfangantikörper umfasst, der gegen eine erste Variante von hCG spezifisch ist, wobei das Vorliegen der ersten hCG-Variante in der Flüssigprobe an der zweiten Einfangstelle durch das Vorliegen eines zweiten nachweisbaren Signals angezeigt wird, das durch das Vorliegen der Sandwich-Komplexe an der zweiten Einfangstelle verursacht wird.

2. Lateral-Flow-Teststreifen nach Anspruch 1, wobei die erste Einfangstelle und die zweite Einfangstelle auf dem ersten chromatographischen Substrat angeordnet sind.

3. Lateral-Flow-Teststreifen nach Anspruch 1, wobei der Lateral-Flow-Test ein erstes Substrat und ein zweites Substrat enthält, wobei das erste Substrat die erste Einfangstelle und das zweite Substrat die zweite Einfangstelle umfasst.

4. Lateral-Flow-Teststreifen nach einem der Ansprüche 1 und 2, wobei erste Einfangstelle und die zweite Einfangstelle auf einem ersten chromatographischen Substrat entlang eines ersten Flüssigprobenfließwegs angeordnet sind, wobei sich der erste Flüssigkeitsfließweg von einer ersten Probenauftragstelle zu sowohl der ersten als auch der zweiten Einfangstelle erstreckt.

5. Lateral-Flow-Teststreifen nach einem der Ansprüche 1 bis 3, wobei durch das erste chromatographische Substrat ziehende Flüssigprobe die zweite Einfangstelle vor Erreichen der ersten Einfangstelle erreicht.

6. Lateral-Flow-Teststreifen nach einem der Ansprüche 1 bis 3, wobei durch das erste chromatographische Substrat ziehende Flüssigprobe die erste Einfangstelle und die zweite Einfangstelle ungefähr zur gleichen Zeit erreicht.

7. Lateral-Flow-Teststreifen nach einem der Ansprüche 1 bis 2, wobei die erste Einfangstelle auf einer ersten Oberfläche des ersten chromatographischen Substrats entlang eines zweiten Flüssigprobenfließwegs angeordnet sind, der sich von der Probenauftragstelle zur ersten Einfangstelle erstreckt, wobei die zweite Einfangstelle auf der ersten Oberfläche des ersten chromatographischen Substrats entlang eines dritten Flüssigprobenfließwegs angeordnet ist, wobei sich der zweite Flüssigprobenfließweg nicht über die zweite Einfangstelle erstreckt, wobei sich der dritte Flüssigprobenfließweg nicht über die erste Einfang-stelle erstreckt.

8. Verfahren zur Bestimmung eines Verhältnisses einer zweiten Konzentration zu einer ersten Konzentration, umfassend:

Betrachten des Lateral-Flow-Teststreifens nach einem der Ansprüche 1 bis 3, nachdem eine Flüssigprobe auf die wenigstens eine Probenauftragstelle aufgetragen wurde, so dass die Flüssigprobe von der wenigstens einen Probenauftragstelle durch das chromatographische Substrat zur ersten Einfangstelle und zur zweiten Einfangstelle gezogen ist;

Erhalten einer ersten Konzentration einer Gruppe von mehreren hCG-Varianten an der ersten Einfangstelle durch Bewerten des ersten nachweisbaren Signals;

Erhalten einer zweiten Konzentration einer ersten hCG-Variante an der zweiten Einfangstelle durch Bewerten des zweiten nachweisbaren Signals; und

Bestimmen des Verhältnisses der zweiten Konzentration zur ersten Konzentration.

9. Verfahren nach Anspruch 8, wobei der Schritt des Erhaltens der ersten Konzentration Vergleichen des ersten nachweisbaren Signals gegen einen ersten Referenzwert umfasst, wobei der erste Referenzwert einer bekannten Konzentration der Gruppe von mehreren hCG-Varianten in einer ersten Referenz-Flüssigprobe in einem entsprechenden Lateral-Flow-Test entspricht; und

wobei der Schritt des Erhaltens der zweiten Konzentration Vergleichen des zweiten nachweisbaren Signals gegen einen zweiten Referenzwert umfasst, wobei der zweite Referenzwert einer bekannten Konzentration der ersten hCG-Variante in einer zweiten Referenz-Flüssigprobe im entsprechenden Lateral-Flow-Test entspricht.

10. Verfahren nach Anspruch 9, wobei durch das erste chromatographische Substrat ziehende Flüssigprobe die zweite Einfangstelle vor Erreichen des ersten Einfangs erreicht und wobei Bestimmen des Verhältnisses der zweiten Konzentration der ersten Variante ($R_1$) in der Flüssigprobe zur ersten Konzentration der Gruppe von hCG-Varianten ($R_2$) in der Flüssigprobe Verwenden der folgenden Gleichung umfasst:

$$(R1)/(R1+R2).$$

11. System, umfassend einen optischen Detektor und ein nichtflüchtiges computerlesbares Medium, das Anweisungen speichert, die bei ihrer Ausführung durch das System veranlassen, dass wenigstens ein Prozessor des Systems die Ergebnisse des Lateral-Flow-Teststreifens nach einem der Ansprüche 1 bis 3, nachdem die Flüssigprobe auf die wenigstens eine Probenauftragstelle aufgetragen wurde, so dass die Flüssigprobe von der wenigstens einen Probenauftragstelle durch das chromatographische Substrat zur ersten Einfangstelle und zur zweiten Einfangstelle gezogen ist, interpretiert, indem:

die Konzentration einer Gruppe von mehreren hCG-Varianten an der ersten Einfangstelle und die Konzentration einer ersten Variante an der zweiten Einfangstelle durch Erhalten des entsprechenden ersten nachweisbaren Signals bzw. zweiten nachweisbaren Signals erfasst wird, wobei das erste nachweisbare Signal und das zweite nachweisbare Signal durch Messen der Reflektanz der ersten Einfangstelle bzw. der zweiten Einfangstelle mit dem optischen Detektor erhalten werden; und

das Verhältnis der Konzentration der ersten hCG-Variante in der Flüssigprobe zur Konzentration der Gruppe von mehreren hCG-Varianten in der Flüssigprobe bezogen auf die erhaltene Konzentration der Gruppe von mehreren hCG-Varianten an der ersten Einfangstelle und die Konzentration der ersten hCG-Variante an der zweiten Einfangstelle bestimmt wird.

12. System nach Anspruch 11, wobei der Schritt des Erhaltens der Konzentration der Gruppe von hCG-Varianten an der ersten Einfangstelle Vergleichen des ersten nachweisbaren Signals gegen einen ersten Referenzwert umfasst, wobei der erste Referenzwert einer bekannten Konzentration der Gruppe von hCG-Varianten in einer ersten Referenz-Flüssigprobe entspricht; und

wobei der Schritt des Erhaltens der Konzentration der ersten hCG-Variante an der zweiten Einfangstelle Vergleichen des zweiten nachweisbaren Signals gegen einen zweiten Referenzwert umfasst, wobei der zweite Referenzwert einer bekannten Konzentration der ersten hCG-Variante in einer zweiten Referenz-Flüssigprobe entspricht.

13. System nach Anspruch 12, wobei durch das erste chromatographische Substrat ziehende Flüssigprobe die zweite Einfangstelle vor Erreichen der ersten Einfangstelle erreicht (z. B. die Vorrichtung nach Anspruch 4) und wobei Bestimmen des Verhältnisses der Konzentration der ersten Variante ($R_1$) in der Flüssigprobe zur Gesamtkonzentration der Gruppe von hCG-Varianten ($R_2$) in der Flüssigprobe Verwenden der folgenden Gleichung umfasst:

$$(R1)/(R1+R2).$$

**Revendications**

1. Bandelette de dosage à écoulement latéral comprenant :

   un ou plusieurs substrats chromatographiques ;
   un site d'application d'échantillon sur chaque substrat chromatographique pour l'application d'une partie d'un échantillon liquide ;
   un premier site de capture disposé sur un premier substrat chromatographique, le premier site de capture étant espacé du site d'application de telle sorte que lorsque l'échantillon liquide est appliqué sur le site d'application d'échantillon, l'échantillon liquide se déplace à travers le premier substrat chromatographique du site d'application d'échantillon au premier site de capture, le premier site de capture comprenant un anticorps de capture anti-gonadotrophine chorionique humaine (« hCG ») ayant une large affinité pour une grappe de multiples variants de l'hCG, la présence de la grappe de variants de l'hCG dans l'échantillon liquide étant indiquée au niveau du premier site de capture par la présence d'un premier signal détectable provoqué par la présence de complexes en sandwich au niveau du premier site de capture ; et
   un deuxième site de capture disposé sur le premier substrat chromatographique ou un deuxième substrat chromatographique, le deuxième site de capture étant espacé du site d'application d'échantillon de sorte que lorsque l'échantillon liquide est appliqué au site d'application d'échantillon, l'échantillon liquide se déplace à travers le premier substrat chromatographique ou un deuxième substrat chromatographique du site d'application d'échantillon au deuxième site de capture, le deuxième site de capture comprenant un anticorps de capture anti-hCG qui est spécifique d'un premier variant de l'hCG, la présence du premier variant de l'hCG dans l'échantillon liquide étant indiquée au niveau du deuxième site de capture par la présence d'un deuxième signal détectable provoqué par la présence des complexes en sandwich au niveau du deuxième site de capture.

2. Bandelette de dosage à écoulement latéral selon la revendication 1, dans laquelle le premier site de capture et le deuxième site de capture sont disposés sur le premier substrat chromatographique.

3. Bandelette de dosage à écoulement latéral selon la revendication 1, dans laquelle le dosage à écoulement latéral contient un premier substrat et un deuxième substrat, le premier substrat comprenant le premier site de capture et le deuxième substrat comprenant le deuxième site de capture.

4. Bandelette de dosage à écoulement latéral selon l'une quelconque des revendications 1 et 2, dans laquelle le premier site de capture et le deuxième site de capture sont disposés sur un premier substrat chromatographique le long d'un premier trajet d'écoulement d'échantillon liquide, le premier trajet d'écoulement liquide s'étendant d'un premier site d'application d'échantillon vers les premier et deuxième sites de capture.

5. Bandelette de dosage à écoulement latéral selon l'une quelconque des revendications 1 à 3, dans laquelle l'échantillon liquide se déplaçant à travers le premier substrat chromatographique atteint le deuxième site de capture avant d'atteindre le premier site de capture.

6. Bandelette de dosage à écoulement latéral selon l'une quelconque des revendications 1 à 3, dans laquelle l'échantillon liquide se déplaçant à travers le premier substrat chromatographique atteint le premier site de capture et le deuxième site de capture approximativement en même temps.

7. Bandelette de dosage à écoulement latéral selon l'une quelconque des revendications 1 à 2, dans laquelle le premier site de capture est disposé sur une première surface du premier substrat chromatographique le long d'un deuxième trajet d'écoulement d'échantillon liquide s'étendant du site d'application d'échantillon au premier site de capture, dans laquelle le deuxième site de capture est disposé sur la première surface du premier substrat chromatographique le long d'un troisième trajet d'écoulement d'échantillon liquide, dans laquelle le deuxième trajet d'écoulement d'échantillon liquide ne s'étend pas à travers le deuxième site de capture, dans laquelle le troisième trajet d'écoulement d'échantillon liquide ne s'étend pas à travers le premier site de capture.

8. Procédé de détermination d'un rapport entre une deuxième concentration et une première concentration, comprenant les étapes consistant à :

   observer la bandelette de dosage à écoulement latéral selon l'une quelconque des revendications 1 à 3 après qu'un échantillon liquide a été appliqué audit au moins un site d'application d'échantillon de sorte que l'échantillon liquide s'est déplacé à travers le substrat chromatographique depuis ledit au moins un site d'application d'échantillon vers le premier site de capture et le deuxième site de capture ;
obtenir une première concentration d'un groupe de variants multiples de l'hCG au niveau du premier site de capture en évaluant le premier signal détectable ;
obtenir une deuxième concentration d'un premier variant de l'hCG au niveau du deuxième site de capture en évaluant le deuxième signal détectable; et
déterminer le rapport entre la deuxième concentration et la première concentration.

**9.** Procédé selon la revendication 8 dans lequel l'étape consistant à obtenir la première concentration comprend la comparaison du premier signal détectable à une première valeur de référence, la première valeur de référence correspondant à une concentration connue du groupe de variants de l'hCG multiples dans un premier échantillon liquide de référence dans un dosage à écoulement latéral correspondant ; et
dans lequel l'étape consistant à obtenir la deuxième concentration comprend la comparaison du deuxième signal détectable à une deuxième valeur de référence, la deuxième valeur de référence correspondant à une concentration connue de la première variant de l'hCG dans un deuxième échantillon liquide de référence dans le dosage à écoulement latéral correspondant.

**10.** Procédé selon la revendication 9, dans lequel l'échantillon liquide se déplaçant à travers le premier substrat chromatographique atteint le deuxième site de capture avant d'atteindre la première capture, et dans lequel la détermination du rapport de la deuxième concentration du premier variant ($R_1$) dans l'échantillon liquide sur la première concentration du groupe de variants de l'hCG ($R_2$) dans l'échantillon liquide comprend l'utilisation de l'équation :

$$(R1)/(R1+R2)$$

**11.** Système comprenant un détecteur optique et un support lisible par ordinateur non transitoire qui stocke des instructions qui, lorsqu'elles sont exécutées par le système, amènent le système ayant au moins un processeur à interpréter les résultats de la bandelette de dosage à écoulement latéral selon l'une quelconque des revendications 1 à 3, après que l'échantillon liquide a été appliqué audit au moins un site d'application d'échantillon de sorte que l'échantillon liquide s'est déplacé à travers le substrat chromatographique depuis ledit au moins un site d'application d'échantillon vers le premier site de capture et le deuxième site de capture, par :

l'acquisition de la concentration d'un groupe de variants multiples de l'hCG au niveau du premier site de capture et de la concentration d'un premier variant au niveau du deuxième site de capture en obtenant le premier signal détectable et le deuxième signal détectable respectifs, dans lequel le premier signal détectable et le deuxième signal détectable sont obtenus en mesurant la réflectance du premier site de capture et du deuxième site de capture, respectivement, avec le détecteur optique ; et
la détermination du rapport de la concentration du premier variant de l'hCG dans l'échantillon liquide sur la concentration du groupe de multiples variants de l'hCG dans l'échantillon liquide sur la base de la concentration obtenue du groupe de multiples variants de l'hCG au niveau du premier site de capture et de la concentration du premier variant de l'hCG au niveau du deuxième site de capture.

**12.** Système selon la revendication 11, dans lequel l'étape consistant à obtenir la concentration du groupe de variants de l'hCG au niveau du premier site de capture comprend la comparaison du premier signal détectable à une première valeur de référence, la première valeur de référence correspondant à une concentration connue du groupe de variants de l'hCG dans un premier échantillon liquide de référence ; et
dans lequel l'étape consistant à obtenir la concentration du premier variant de l'hCG au deuxième site de capture comprend la comparaison du deuxième signal détectable à une deuxième valeur de référence, la deuxième valeur de référence correspondant à une concentration connue du premier variant de l'hCG dans un deuxième échantillon liquide de référence.

**13.** Système selon la revendication 12, dans lequel l'échantillon liquide se déplaçant à travers le premier substrat chromatographique atteint le deuxième site de capture avant d'atteindre le premier site de capture (par exemple, le dispositif selon la revendication 4), et dans lequel la détermination du rapport de la concentration du premier variant ($R_1$) dans l'échantillon liquide sur la concentration totale du groupe de variants de l'hCG ($R_2$) dans l'échantillon liquide comprend l'utilisation de l'équation :

$$(R1)/(R1+R2)$$

Fig. 1A

Fig. 1B

Fig. 2

Fig. 3

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007172963 A